# FASCICULE DE BREVET EUROPEEN

(11) **EP 2 246 339 B1**
(45) Date de publication et mention de la délivrance du brevet: **22.06.2011**
(21) Numéro de dépôt: 10290141.0
(22) Date de dépôt: 18.03.2010
(51) Int. Cl.: C07D 285/36, A61K 31/554, A61P 25/00

(54) **Dérivés de benzothiazépine et leur utilisation en tant que modulateurs des récepteurs AMPA et NMDA**
Benzothiazepinderivate und ihre Verwendung als Modulatoren von AMPA und NMDA Rezeptoren
Derivatives of benzothiazepine and their use as modulators of AMPA and NMDA receptors

(30) Priorité: 20.03.2009 FR 0901300
(43) Date de publication de la demande: 03.11.2010
(73) Titulaire: Les Laboratoires Servier, 92284 Suresnes Cedex (FR)
(72) Inventeur: Cordi, Alexis, 92150 Suresnes (FR); Desos, Patrice, 92270 Bois-Colombes (FR); Lestage, Pierre, 78170 La Celle Saint Cloud (FR); Danober, Laurence, 78360 Montesson (FR)

(56) Documents cités:
- EP-A- 1 655 030
- WO-A-93/21170
- WO-A-99/41246
- WO-A-03/091232
- US-A- 6 156 746

## Description

La présente invention concerne de nouveaux dérivés de benzothiadiazépines, leur procédé de préparation, les compositions pharmaceutiques qui les contiennent ainsi que leur utilisation en tant que modulateurs allostériques positifs des récepteurs AMPA et antagonistes des récepteurs NMDA.

Il est désormais reconnu que les aminoacides excitateurs, et tout particulièrement le glutamate, jouent un rôle crucial dans les processus physiologiques de plasticité neuronale et dans les mécanismes sous-tendant l'apprentissage et la mémoire. Des études pathophysiologiques ont indiqué clairement qu'un déficit de la neurotransmission glutamatergique est étroitement associé avec le développement de la maladie d'Alzheimer (Neuroscience and Biobehavioral Reviews, 1992, 16, 13-24 ; Progress in Neurobiology, 1992, 39, 517-545).

Par ailleurs, de nombreux travaux ont démontré l'existence de sous-types réceptoriels aux aminoacides excitateurs et de leurs interactions fonctionnelles (Molecular Neuropharmacology, 1992, 2, 15-31).

Parmi ces récepteurs, le récepteur à l'AMPA ("α-amino-3-hydroxy-5-methyl-4-isoxazole-propionic acid") est un récepteur-canal au glutamate, perméable au Na⁺ et au K⁺ et peu perméable au Ca²⁺. Le récepteur AMPA apparaît être le plus impliqué dans la transmission synaptique excitatrice physiologique rapide et notamment dans les processus de mémorisation. Pour exemple, l'apprentissage a été montré comme étant associé à une augmentation du nombre de récepteurs AMPA au niveau des contacts synaptiques dans l'hippocampe, l'une des zones cérébrales essentielles aux processus mnémocognitifs. De même, les agents nootropes tels que l'aniracetam ont été décrits comme modulant positivement les récepteurs AMPA des cellules neuronales (J. Neurochemistry, 1992, 58, 1199-1204).

Un second récepteur, le récepteur au NMDA (N-méthyl-D-aspartate), appartient lui aussi à l'une des trois classes de récepteur-canaux au glutamate. La liaison du glutamate et de son co-agoniste, la glycine, est nécessaire pour conduire à l'ouverture du canal et permettre l'entrée de Ca²⁺ dans la cellule (Progress in Neurobiology, 1998, 54, 581-618 ; Handb. Exp. Pharmacol., 2005, 169, 249-303). Les récepteurs NMDA ont également un rôle physiologique important dans l'apprentissage et la mémoire. Néanmoins, une activation excessive de ces récepteurs, qui surviendrait lors d'ischémie et anoxie cérébrales, de crises d'épilepsie, de trauma ou de maladies neurodégénératives, conduit à une entrée excessive de Ca²⁺ dans la cellule qui serait susceptible d'entraîner la mort de celle-ci par un phénomène d'excitotoxicité (Progress in Neurobiology, 1998, 54, 369-415 ; J. Pharmacol. Exp. Ther., 2002, 300, 717-723). En effet, il a été montré que certaines formes de neurotoxicité induite au glutamate sont dépendantes d'une accumulation de Ca²⁺ dans la cellule, qui conduit à une augmentation du stress métabolique des mitochondries et à l'augmentation de la production de radicaux libres. Pour ces raisons, des antagonistes des récepteurs NMDA ont été développés pour leurs propriétés neuroprotectrices, dont la mémantine qui est actuellement indiquée dans le traitement des formes modérément sévères à sévères de la maladie d'Alzheimer (Neuropharmacology, 1999, 38, 735-767; Dement. Geriatr. Cogn. Disord., 2005, 19, 229- 245).

Dans la littérature, très peu de composés de structure benzothiadiazine ont été décrits comme étant des modulateurs positifs des récepteurs AMPA ayant aussi une activité antagoniste du récepteur NMDA (Neuropharmacology, 2004, 46, 1105-1113). Le composé IDRA-21, en particulier, est capable d'améliorer les performances mnésiques.

La demande de brevet WO 99/42456 décrit, entre autres, des dérivés de benzothiadiazine et de benzothiadiazépine en tant que modulateurs des récepteurs AMPA.

Les dérivés de benzothiadiazépines, objets de la présente invention, outre le fait qu'ils soient nouveaux, présentent des activités pharmacologiques à la fois pour le récepteur AMPA et pour le récepteur NMDA, supérieures à celles des composés de structures proches décrits dans l'Art Antérieur.

Plus spécifiquement, la présente invention concerne les composés de formule (I) : 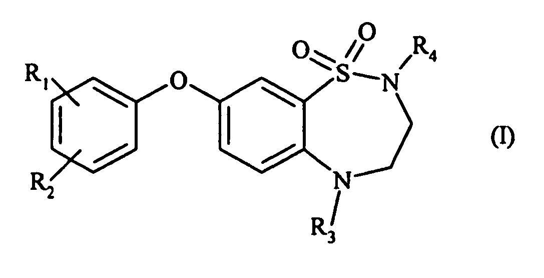 dans laquelle :
➢ R₁ et R₂, identiques ou différents, représentent chacun, un atome d'hydrogène ; un atome d'halogène ; un groupement alkyle (C₁-C₆) linéaire ou ramifié non-substitué ou substitué par un ou plusieurs atomes d'halogène ; un groupement alkoxy (C₁-C₆) linéaire ou ramifié ; un groupement alkylthio (C₁-C₆) linéaire ou ramifié ; un groupement alkoxycarbonyle (C₁-C₆) linéaire ou ramifié ; un groupement carboxy ; un groupement acyle (C₁-C₆) linéaire ou ramifié ; un groupement hydroxy ; un groupement hydroxyalkyle (C₁-C₆) linéaire ou ramifié ; un groupement cyano ; un groupement nitro ; un groupement amino non-substitué ou substitué par un ou plusieurs groupements alkyle (C₁-C₆) linéaire ou ramifié ; un groupement amino substitué par un groupement acyle (C₁-C₆) linéaire ou ramifié ; un groupement aminocarbonyle non-substitué ou substitué par un ou plusieurs groupements alkyle (C₁-C₆) linéaire ou ramifié ; un groupement aminosulfonyle non-substitué ou substitué par un ou plusieurs groupements alkyle (C₁-C₆) linéaire ou ramifié ; un groupement alkyl(C₁-C₆)sulfonylaminoalkyle (C₁-C₆) linéaire ou ramifié ; un groupement N-hydroxycarboximidamide ou un groupement benzyloxy,
➢ R₃ représente un atome d'hydrogène, un groupement alkyle (C₁-C₆) linéaire ou ramifié, un groupement cycloalkyle (C₃-C₈), ou un groupement cycloalkyl(C₃-C₈)alkyle (C₁-C₆) linéaire ou ramifié,
➢ R₄ représente un atome d'hydrogène ou un groupement alkyle (C₁-C₆) linéaire ou ramifié non-substitué ou substitué par un ou plusieurs atomes d'halogène,
leurs isomères optiques et de position, lorsqu'ils existent, ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphorique, acétique, trifluoroacétique, lactique, pyruvique, malonique, succinique, glutarique, fumarique, tartrique, maléique, citrique, ascorbique, oxalique, méthanesulfonique, benzènesulfonique, camphorique.

Parmi les bases pharmaceutiquement acceptables, on peut citer à titre non limitatif l'hydroxyde de sodium, l'hydroxyde de potassium, la triéthylamine, la tertbutylamine.

Le groupement R₁ représente préférentiellement un atome d'hydrogène ou le groupement hydroxy; le groupement hydroxyalkyle (C₁-C₆) linéaire ou ramifié, et plus particulièrement le groupement hydroxyméthyle ; le groupement alkoxycarbonyle (C₁-C₆) linéaire ou ramifié, et avantageusement le groupement éthoxycarbonyle ; le groupement amino, ou le groupement aminocarbonyle substitué par un groupement alkyle (C₁-C₆) linéaire ou ramifié, et plus particulièrement le groupement *N*-méthyl-aminocarbonyle. De manière préférée, le groupement R₁ se trouve en position méta ou para. D'une façon particulièrement avantageuse, R₁ représente un atome d'hydrogène.

Les groupements R₂ et R₄ préférés sont l'atome d'hydrogène.

R₃ représente préférentiellement un atome d'hydrogène ou un groupement méthyle.

Les composés préférés de l'invention sont le :
- 8-phénoxy-2,3,4,5-tétrahydro-1,2,5-benzothiadiazépine 1,1-dioxyde ;
- 3-[(1,1-dioxydo-2,3,4,5-tétrahydro-1,2,5-benzothiadiazépin-8-yl)oxy]benzoate d'éthyle ;
- 3-[(1,1-dioxydo-2,3,4,5-tétrahydro-1,2,5-benzothiadiazépin-8-yl)oxy]-*N*-méthyl benzamide ;
- {3-[(1,1-dioxydo-2,3,4,5-tétrahydro-1,2,5-benzothiadiazépin-8-yl)oxy]phényl} méthanol ;
- 4-[(1,1-dioxydo-2,3,4,5-tétrahydro-1,2,5-benzothiadiazépin-8-yl)oxy]phénol ;
- 4-[(5-méthyl-1,1-dioxydo-2,3,4,5-tétrahydro-1,2,5-benzothiadiazépin-8-yl)oxy] phénol ;
- 4-[(1,1-dioxydo-2,3,4,5-tétrahydro-1,2,5-benzothiadiazépin-8-yl)oxy]aniline.

Les sels d'addition à un acide ou une base pharmaceutiquement acceptable des composés préférés de l'invention font partie intégrante de l'invention.

L'invention s'étend également au procédé de préparation des composés de formule (I), caractérisé en ce que l'on utilise comme produit de départ un composé de formule (II) : 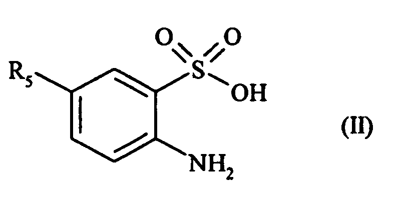 dans laquelle R₅ représente un groupement alkoxy (C₁-C₆) linéaire ou ramifié,
qui est mis en réaction avec le chlorure de thionyle en présence de diméthylformamide, pour conduire au composé de formule (III) : 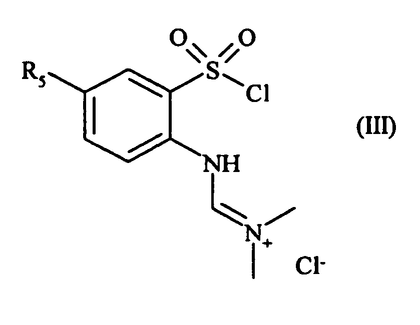 dans laquelle R₅ est tel que défini précédemment,
qui subit alors l'action de la 2-chloroéthylamine en milieu basique, pour conduire au composé de formule (IV) : 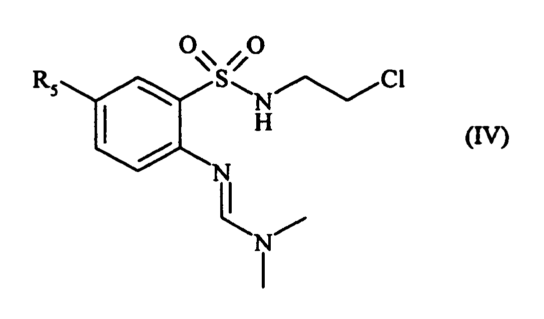 dans laquelle R₅ est tel que défini précédemment,
qui, après déprotection en milieu acide, est ensuite cyclisé pour conduire au composé de formule (V) : 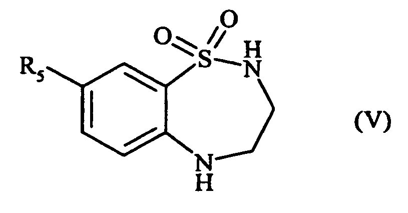 dans laquelle R₅ est tel que défini précédemment,
qui subit alors l'action du tribromure de bore, pour conduire au composé de formule (VI) : 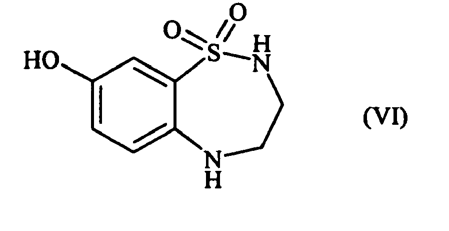 que l'on fait réagir avec un dérivé d'acide boronique de formule (VII) : 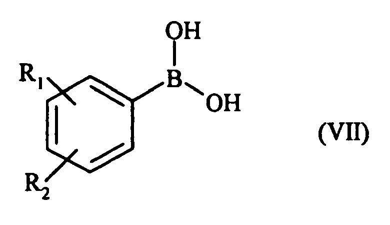 dans laquelle R₁ et R₂ ont la même signification que dans la formule (I),
pour conduire au composé de formule (I/a), cas particulier des composés de formule (I) : 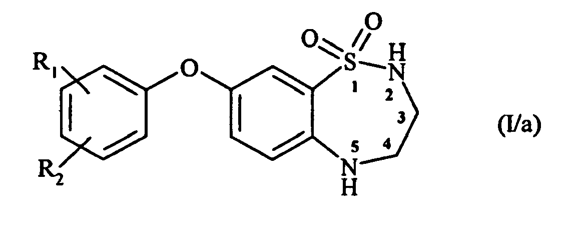 dans laquelle R₁ et R₂ sont tels que définis précédemment,
une variante dans la préparation du composé de formule (I/a) consistant, une fois l'étape de couplage sur le composé de formule (VI) réalisée, en l'utilisation des réactions classiques de chimie afin de modifier, dans un deuxième temps, les substituants du dérivé de l'acide boronique,
composé de formule (I/a) qui peut alors subir, si nécessaire :
■ **soit** une double alkylation sur les azotes en positions 2 et 5, par action d'une base forte en présence d'un agent d'alkylation R'-X dans lequel R' représente un groupement alkyle (C₁-C₆) linéaire ou ramifié et X représente un atome d'halogène, pour conduire au composé de formule (I/b), cas particulier des composés de formule (I) : 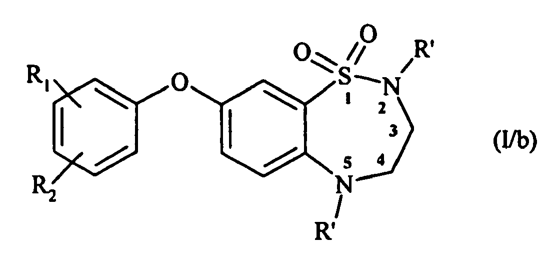 dans laquelle R₁, R₂ et R' sont tels que définis précédemment,
■ **soit** une alkylation sur l'azote en position 2, par action d'une base en présence d'un agent d'alkylation R'₄-X dans lequel R'₄ représente un groupement alkyle (C₁-C₆) linéaire ou ramifié non-substitué ou substitué par un ou plusieurs atomes d'halogène et X représente un atome d'halogène,
   pour conduire au composé de formule (I/c), cas particulier des composés de formule (I) : 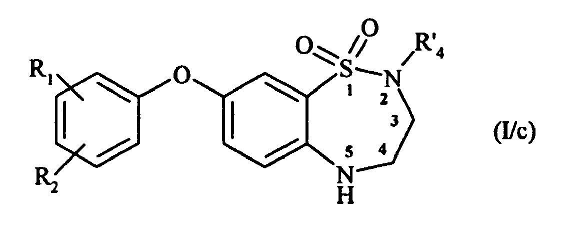 dans laquelle R₁, R₂ et R'₄ sont tels que définis précédemment,
   qui peut, éventuellement, subir une alkylation sur l'azote en position 5, par action d'une base en présence d'un agent d'alkylation R'₃-X dans lequel R'₃ représente un groupement alkyle (C₁-C₆) linéaire ou ramifié, un groupement cycloalkyle (C₃-C₈) ou un groupement cycloalkyl(C₃-C₈)alkyle(C₁-C₆) linéaire ou ramifié, et X représente un atome d'halogène,
   pour conduire au composé de formule (I/d) : 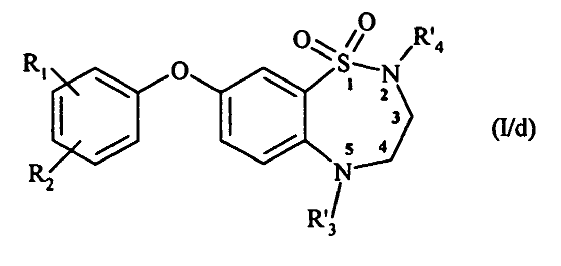 dans laquelle R₁, R₂, R'₃ et R'₄ sont tels que définis précédemment,
■ **soit** une alkylation sur l'azote en position 5, par une réaction d'amination réductrice avec un agent réducteur, tel que le triacétoxyborohydrure ou le cyanoborohydrure de sodium, en présence :
   - soit du [(1-éthoxycyclopropyl)oxy]triméthylsilane,
   - soit d'un composé de formule (VIII) :

      R"₃-CHO (VIII)

      dans laquelle R"₃ représente un atome d'hydrogène ou un groupement alkyle (C₁-C₅) linéaire ou ramifié, un groupement cycloalkyle (C₃-C₈) ou cycloalkyl(C₃-C₈)alkyle (C₁-C₅) linéaire ou ramifié,
   - soit d'un composé de formule (IX) : 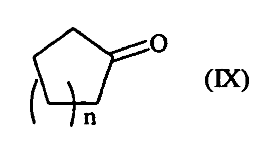 dans laquelle 0 ≤ n ≤ 4,
pour conduire au composé de formule (I/e), cas particulier des composés de formule (I) : 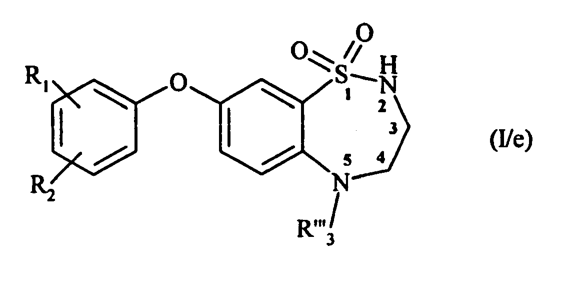 dans laquelle R"'₃ représente un groupement alkyle (C₁-C₆) linéaire ou ramifié, cycloalkyle (C₃-C₈) ou cycloalkyl(C₃-C₈)alkyle (C₁-C₆) linéaire ou ramifié, et R₁ et R₂ sont tels que définis précédemment,
qui peut, éventuellement, subir une alkylation sur l'azote en position 2, par action d'une base en présence d'un agent d'alkylation R"₄-X dans lequel R"₄ représente un groupement alkyle (C₁-C₆) linéaire ou ramifié non-substitué ou substitué par un ou plusieurs atomes d'halogène et X représente un atome d'halogène,
pour conduire au composé de formule (I/f) : 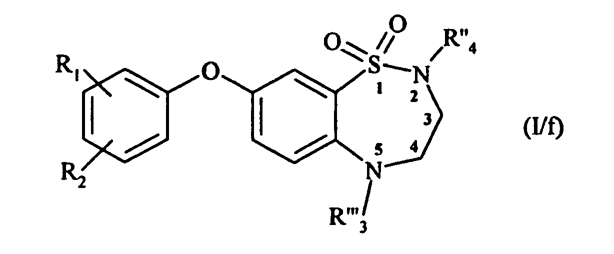 dans laquelle R₁, R₂, R"'₃ et R"₄ sont tels que définis précédemment,
les composés de formule (I/a) à (I/f), qui constituent l'ensemble des composés de formule (I), peuvent être ensuite purifiés selon une technique classique de séparation, que l'on transforme, si on le souhaite, en ses sels d'addition à un acide ou à une base pharmaceutiquement acceptable et dont on sépare éventuellement les isomères optiques et de position, s'ils existent, selon une technique classique de séparation.

Les composés de formule (II) et (VII) sont commerciaux ou aisément accessibles à l'homme du métier par des réactions de chimie classiques ou décrites dans la littérature.

Le composé de formule (VI) est nouveau et fait également partie de l'invention à titre d'intermédiaire de synthèse des composés de formule (I).

Les composés de formule (I) selon l'invention présentent des propriétés activatrices des récepteurs AMPA et antagonistes des récepteurs NMDA qui les rendent utiles dans le traitement ou la prévention des maladies neurodégénératives progressives, de la maladie d'Alzheimer, de la maladie de Parkinson, de la maladie de Pick, de la chorée d'Huntington, de la maladie de Korsakoff, de la schizophrénie, des maladies neurodégénératives aiguës, des démences frontales et sous-corticales, des démences vasculaires, de l'épilepsie, des accidents vasculaires cérébraux ainsi que des états dépressifs et anxieux.

L'invention s'étend aussi aux compositions pharmaceutiques renfermant comme principe actif au moins un composé de formule (I) avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, percutanée, transcutanée, rectale, perlinguale, oculaire ou respiratoire et, notamment, les comprimés simples ou dragéifiés, les comprimés sublinguaux, les sachets, les paquets, les gélules, les tablettes, les glossettes, les suppositoires, les crèmes, les pommades, les gels dermiques, les préparations injectables, et les suspensions buvables.

La posologie utile est adaptable selon la nature et la sévérité de l'affection, la voie d'administration ainsi que l'âge et le poids du patient. Cette posologie varie de 0,01 mg à 1 g par jour en une ou plusieurs prises.

Les exemples suivants illustrent l'invention mais ne la limitent en aucune façon.

Les produits de départ utilisés sont des produits connus ou préparés selon des modes préparatoires connus.

Les structures des composés décrits dans les exemples ont été déterminées selon les techniques spectrophotométriques usuelles (infrarouge, RMN, spectrométrie de masse).

### EXEMPLE 1 : 8-phénoxy-2,3,4,5-tétrahydro-1,2,5-benzothiadiazépine 1,1-dioxyde

### Stade A : Chlorure de N-[[[2-(chlorosu/fonyl)-4-méthoxyphény/]amino] méthylène]-N-méthyl-méthanaminium

A une suspension d'acide 2-amino-5-méthoxybenzènesulfonique (0,152 mol) dans du chlorure de thionyle (0,763 mol), est ajouté goutte à goutte du diméthylformamide (0,152 mol). Le milieu réactionnel est chauffé très progressivement à 70 °C et est maintenu à cette température pendant 1,5 heures. Après refroidissement, la solution réactionnelle sous agitation est traitée par addition de 20 ml de toluène. Il se forme un précipité qui est rapidement filtré, rincé au toluène puis séché à 40 °C sous vide en présence de pastilles de KOH, correspondant au produit du titre.

### Stade B : N-(2-chloroéthyl)-2-{[(1E)-(diméthylamino)méthylène]amino}-5-méthoxybenzènesulfonamide

A une suspension d'un mélange de produit du stade précédent (0,152 mol) et de chlorhydrate de 2-chloroéthylamine (0,182 mol) dans 470 ml de dichlorométhane, est ajoutée goutte à goutte la triéthylamine (0,517 mol) en maintenant la température du milieu réactionnel inférieure à 30 °C. Après 2 heures d'agitation, la réaction est diluée dans du dichlorométhane et la phase organique est lavée deux fois à l'eau puis par une solution saturée en NaCl. La phase organique est séchée sur MgSO₄ et évaporée sous pression réduite. Le résidu est concrétisé dans l'éther éthylique pour conduire, après filtration, au produit du titre.

### Point de fusion : 89 °C

### Microanalyse élémentaire :

| | *C* | *H* | *N* | *S* | *Cl* |
|---|---|---|---|---|---|
| *% théorique* | *45, 07* | *5, 67* | *13,14* | *10, 03* | *11,09* |
| *% expérimental* | *44,93* | *5,63* | *13, 07* | *10,19* | *11,82* |

### Stade C : 2-amino-N-(2-chloroéthyl)-5-méthoxybenzènesulfonamide

La totalité du produit du stade précédent est mise en suspension dans un mélange de 130 ml de dioxane et 110 ml de HCl 5 M. Le milieu réactionnel est agité à 110 °C pendant 16 heures. Après évaporation du dioxane, le milieu réactionnel est dilué dans l'eau puis est neutralisé par addition d'une solution à 10 % en NaHCO₃. A pH neutre, la phase aqueuse est extraite 3 fois par l'acétate d'éthyle. Les phases organiques sont rassemblées, lavées par une solution saturée en NaCl, puis séchées sur MgSO₄ pour conduire, après évaporation sous pression réduite, au produit du titre sous forme d'une huile.

### Stade D : 8-méthoxy-2,3,4,5-tétrahydro-1,2,5-benzothiadiazépine 1,1-dioxyde

Dans un réacteur autoclave PAAR de 1 L avec enveloppe de verre, est mise en suspension la totalité du produit du stade précédent dans 300 ml d'éthanol. La réaction est portée à 150 °C pendant 8 heures. Après complet refroidissement, le solide est filtré pour conduire au produit du titre.

### Point de fusion : 126 °C

### Stade E: 2,3,4,5-tétrahydro-1,2,5-benzothiadiazépin-8-ol 1,1-dioxyde

A une suspension du produit du stade précédent (12,3 mmol) dans 30 ml de dichlorométhane, est ajoutée goutte à goutte une solution de tribromure de bore 1 M dans du dichlorométhane (37 ml) en maintenant la température du milieu réactionnel inférieure à 30 °C. Le milieu hétérogène est agité 20 heures à température ambiante et est versé sur environ 100 g de glace. La solution est ensuite neutralisée par addition d'une solution à 10 % en Na₂CO₃. Après évaporation à sec de la phase aqueuse, le résidu est trituré dans l'acétone. Les sels sont filtrés plusieurs fois et les différents filtrats (contenant le produit attendu ainsi que du produit de départ) sont rassemblés. Ces derniers sont absorbés sur silice pour donner, après chromatographie en éluant par un gradient dichlorométhane / acétone de 96 / 4 à 90 / 10 et concrétisation dans l'éther éthylique, le produit du titre sous forme d'une poudre blanche.

### Point de fusion : 183-188 °C

### Stade F : 8-phénoxy-2,3,4,5-tétrahydro-1,2,5-benzothiadiazépine 1,1-dioxyde

Une suspension d'acide phénylboronique (8,36 mmol), de produit du stade précédent (5,60 mmol), de Cu(OAc)₂ (8,42 mmol), de pyridine (16,8 mmol) et 12 g de tamis moléculaire dans 200 ml de dichlorométhane est agitée une nuit à l'air à température ambiante. De l'acétone est ensuite additionnée puis le milieu réactionnel est filtré sur fritté. Le filtrat est évaporé, repris dans du dichlorométhane et chromatographié sur colonne de silice en éluant avec un mélange dichlorométhane / acétone 96 / 4 pour donner, après concrétisation dans l'éther éthylique, le produit du titre sous forme d'une poudre blanche.

### Point de fusion : 146-149 °C

### Microanalyse élémentaire :

| | *C* | *H* | *N* | *S* |
|---|---|---|---|---|
| *% théorique* | *57,92* | *4,56* | *9, 65* | *11, 04* |
| *% expérimental* | *57,90* | *5,00* | *9,78* | *11,08* |

Les produits des Exemples **2-9** suivants ont été obtenus selon le mode opératoire du stade F de l'Exemple 1 à partir de l'intermédiaire 2,3,4,5-tétrahydro-1,2,5-benzothiadiazépin-8-ol 1,1-dioxyde (stade E de l'Exemple 1) et de l'acide boronique approprié comme indiqué.

### EXEMPLE 2: N-{4-[(1,1-dioxydo-2,3,4,5-tétrahydro-1,2,5-benzothiadiazépin-8-yl) oxy]benzyl}méthanesulfonamide

Réaction avec l'acide (4-{[(méthylsulfonyl)amino]méthyl}phényl)boronique.

### Point de fusion : 115-117 °C

### Microanalyse élémentaire :

| | *C* | *H* | *N* | *S* |
|---|---|---|---|---|
| *% théorique* | *48,35* | *4,82* | *10,57* | *16,13* |
| *% expérimental* | *48, 60* | *4,93* | *10,50* | *16, 20* |

### EXEMPLE 3 : 8-(4-fluoro-phénoxy)-2,3,4,5-tétrahydro-1,2,5-benzothiadiazépine 1,1-dioxyde

Réaction avec l'acide (4-fluorophényl)boronique.

### Point de fusion : 153-156 °C

### Microanalyse élémentaire :

| | *C* | *H* | *N* | *S* |
|---|---|---|---|---|
| *% théorique* | *54,54* | *4,25* | *9,09* | *10,40* |
| *% expérimental* | *54,14* | *4,41* | *9,04* | *10,08* |

### EXEMPLE 4 : 8-(3-fluorophénoxy)-2,3,4,5-tétrahydro-1,2,5-benzothiadiazépine 1,1-dioxyde

Réaction avec l'acide (3-fluorophényl)boronique.

### Point de fusion : 128 °C

### Microanalyse élémentaire :

| | *C* | *H* | *N* | *S* |
|---|---|---|---|---|
| *% théorique* | *54,54* | *4,25* | *9,09* | *10,40* |
| *% expérimental* | *54,39* | *4,17* | *8,99* | *9,98* |

### EXEMPLE 5 : 8-(3,5-difluorophénoxy)-2,3,4,5-tétrahydro-1,2,5-benzothiadiazépine 1,1-dioxyde

Réaction avec l'acide (3,5-difluorophényl)boronique.

### Point de fusion : 90-95 °C

### Microanalyse élémentaire :

| | *C* | *H* | *N* | *S* |
|---|---|---|---|---|
| *% théorique* | *51,53* | *3,71* | *8,58* | *9,83* |
| *% expérimental* | *51,44* | *3,86* | *8,50* | *9, 65* |

### EXEMPLE 6 : 3-[(1,1-dioxydo-2,3,4,5-tétr3hydro-1,2,5-benzothiadiazépin-8-yl)oxy] benzonitrile

Réaction avec l'acide (3-cyanophényl)boronique.

### Point de fusion : 172-174 °C

### Microanalyse élémentaire :

| | *C* | *H* | *N* | *S* |
|---|---|---|---|---|
| *% théorique* | *57,13* | *4,15* | *13,33* | *10,17* |
| *% expérimental* | *56,41* | *4,12* | *13,15* | *10, 61* |

### EXEMPLE 7 : 3-[(1,1-dioxydo-2,3,4,5-tétrahydro-1,2,5-benzothiadiazépin-8-yl)oxy] benzoate d'éthyle

Réaction avec l'acide [3-(éthoxycarbonyl)phényl]boronique.

### Point de fusion : 110 °C

### Microanalyse élémentaire :

| | *C* | *H* | *N* | *S* |
|---|---|---|---|---|
| *% théorique* | *56,34* | *5,01* | *7,73* | *8,85* |
| *% expérimental* | *55,70* | *4,95* | *7,76* | *9, 03* |

### EXEMPLE 8 : 8-[4-(benzyloxy)phénoxy]-2,3,4,5-tétrahydro-1,2,5-benzothiadiazépine 1,1-dioxyde

Réaction avec l'acide [4-(benzyloxy)phényl]boronique.

### Point de fusion : 134 °C

### EXEMPLE 9 : 8-(4-nitrophénoxy)-2,3,4,5-tétrahydro-1,2,5-benzothiadiazépine 1,1-dioxyde

Réaction avec l'acide (4-nitrophényl)boronique.

### Point de fusion : 162 °C

### EXEMPLE 10 : 3-[(1,1-dioxydo-2,3,4,5-tétrahydro-1,2,5-benzothiadiazépin-8-yl)oxy]-N'-hydroxybenzènecarboximidamide

Dans 750 µl de DMSO, est ajouté le chlorhydrate d'hydroxylamine (1,90 mmol) puis la triéthylamine (1,90 mmol). Il se forme un abondant précipité blanc qui est dilué en ajoutant 3 ml de THF et l'agitation est poursuivie pendant 25 minutes à température ambiante. Le THF est évaporé sous pression réduite et la suspension est filtrée. Au filtrat, est ajouté le produit de l'Exemple 6 (0,317 mmol) et la solution est agitée une nuit à température ambiante. Après addition d'eau au milieu réactionnel, une gomme se forme puis se solidifie par addition de dichlorométhane et d'éther éthylique. Le solide est filtré, rincé avec de l'eau et de l'éther éthylique pour conduire au produit du titre.

### Point de fusion : 197 °C

### Microanalyse élémentaire :

| | *C* | *H* | *N* | *S* |
|---|---|---|---|---|
| *% théorique* | *51,71* | *4, 63* | *16, 08* | *9, 20* |
| *% expérimental* | *51,38* | *4,58* | *15,61* | *8,84* |

### EXEMPLE 11 : Acide 3-[(1,1-dioxydo-2,3,4,5-tétrahydro-1,2,5-benzothiadiazépin-8-yl)oxy]benzoïque

Une suspension d'ester de l'Exemple 7 (0,55 mmol) dans 5 ml d'une solution de soude 1 N est agitée pendant 30 minutes à 80 °C. Le mélange réactionnel est ensuite neutralisé avec une solution de HCl 1 N, extraite par l'acétate d'éthyle, lavée (solution saturée en NaCl), séchée (MgSO₄), filtrée puis évaporée sous pression réduite pour conduire, après trituration du résidu dans l'éther éthylique et filtration, au produit du titre.

### Point de fusion : 212-217 °C

### Microanalyse élémentaire :

| | *C* | *H* | *N* | *S* |
|---|---|---|---|---|
| *% théorique* | *53,89* | *4, 22* | *8,38* | *9,59* |
| *% expérimental* | *53,41* | *4, 23* | *8,35* | *9,82* |

### EXEMPLE 12 : 3-[(1,1-dioxydo-2,3,4,5-tétrahydro-1,2,5-benzothiadiazépin-8-yl)oxy]-N-méthylbenzamide

L'acide de l'Exemple 11 (0,66 mmol) est agité une nuit à température ambiante dans 50 ml de dichlorométhane en présence de tétrafluoroborate de 2-(1*H*-benzotriazol-1-yl) -1,1,3,3-tétraméthyluronium (0,80 mmol), de diisopropyléthylamine (0,80 mmol) et d'une solution de méthylamine 2 M dans du THF (1,32 mmol). Le milieu réactionnel est ensuite neutralisé avec une solution de HCl 1 N, extrait par l'acétate d'éthyle, lavé (solution saturée en NaCl), séché (MgSO₄), filtré puis évaporé sous pression réduite, pour conduire, après trituration du résidu dans un mélange dichlorométhane / méthanol et filtration, au produit du titre.

### Point de fusion : 205 °C

### Microanalyse élémentaire :

| | *C* | *H* | *N* | *S* |
|---|---|---|---|---|
| *% théorique* | *55,32* | *4,93* | *12,10* | *9, 23* |
| *% expérimental* | *54,79* | *4,88* | *11,88* | *9,20* |

### EXEMPLE 13 : {3-[(1,1-dioxydo-2,3,4,5-tétrahydro-1,2,5-benzothiadiazépin-8-yl)oxy] phényl}méthanol

L'ester de l'Exemple 7 (0,805 mmol) est mis en solution dans 10 ml de THF et le LiAlH₄ (2,43 mmol) est ajouté par petites portions. Après 30 minutes d'agitation, 1 ml d'isopropanol et 1 ml de saumure sont additionnés successivement dans le milieu réactionnel. La suspension est filtrée et le filtrat, après évaporation sous pression réduite, est chromatographié sur colonne de silice (98 / 2 dichlorométhane / méthanol) pour conduire au produit du titre.

### Point de fusion : 146 °C

### EXEMPLE 14 : 4-[(1,1-dioxydo-2,3,4,5-tétrahydro-1,2,5-benzothiadiazépin-8-yl)oxy] phénol

Le produit de l'Exemple 8 (0,76 mmol) est hydrogéné pendant 1 heure à pression atmosphérique dans 60 ml d'éthanol en présence de 30 mg de charbon palladié à 10 % et de 2 gouttes d'une solution de HCl 4 N dans le dioxane. Le catalyseur est éliminé par filtration et le filtrat est évaporé à sec. Le résidu est repris à chaud dans de l'acétate d'éthyle. La phase organique est lavée par une solution à 1 % en NaHCO₃ puis par une solution saturée en NaCl, séchée (MgSO₄), filtrée et évaporée. Le résidu est trituré dans un mélange acétate d'éthyle / éther éthylique pour donner, après filtration, le produit du titre.

### Point de fusion : 175 °C

### Microanalyse élémentaire :

| | *C* | *H* | *N* | *S* |
|---|---|---|---|---|
| *% théorique* | *54,89* | *4, 61* | *9,14* | *10,47* |
| *% expérimental* | *54,96* | *4,74* | *8,89* | *10,39* |

### EXEMPLE 15 : 8-[4-(benzyloxy)phénoxy]-5-méthyl-2,3,4,5-tétrahydro-1,2,5-benzothiadiazépine 1,1-dioxyde

A une suspension du produit de l'Exemple 8 (2,52 mmol) dans 10 ml d'acétonitrile, est ajouté du formaldéhyde aqueux 37 % (12,6 mmol). La suspension est agitée 1 heure à température ambiante. Une pointe de spatule de vert de bromocrésol et du cyanoborohydrure de sodium (7,56 mmol) sont ajoutés successivement. La réaction est amenée à pH acide par addition d'une solution de HCl 4 N dans le dioxane. Le milieu réactionnel est agité une nuit à température ambiante puis est neutralisé par addition d'une solution à 10 % en NaHCO₃. Après dilution du milieu réactionnel dans l'eau, il se forme un précipité qui est filtré. La purification du produit du titre est réalisée par chromatographie sur silice en éluant avec un mélange dichlorométhane / acétate d'éthyle 96/4.

### Point de fusion : 168-172°C

### EXEMPLE 16 : 4-[(5-méthyl-1,1-dioxydo-2,3,4,5-tétrahydro-1,2,5-benzothiadiazépin-8-yl)oxy]phénol

Le composé est obtenu par hydrogénation catalytique du produit de l'Exemple 15 selon le mode opératoire de l'Exemple 14. La purification est réalisée par chromatographie sur silice en éluant avec un mélange dichlorométhane / acétone 95 / 5.

### Point de fusion : 128-130 °C

### Microanalyse élémentaire :

| | *C* | *H* | *N* | *S* |
|---|---|---|---|---|
| *% théorique* | *56, 24* | *5, 03* | *8,74* | *10,01* |
| *% expérimental* | *56,30* | 5*,03* | *8, 67* | *10, 24* |

### EXEMPLE 17 : Dichlorhydrate de 4-[(1,1-dioxydo-2,3,4,5-tétrahydro-1,2,5-benzothiadiazépin-8-yl)oxy] aniline

Le produit de l'Exemple 9 (0,51 mmol) est hydrogéné pendant 2 heures à pression atmosphérique dans 30 ml de méthanol en présence de 20 mg de charbon palladié à 10 %. Le catalyseur est éliminé par filtration et le filtrat est évaporé à sec. Le résidu est repris à chaud dans le méthanol et le mélange est acidifié avec une solution de HCl 4 N dans le dioxane. Après évaporation à sec, le résidu est trituré dans l'acétonitrile pour donner, après filtration, le produit du titre.

### Point de fusion : 128-134 °C

Les produits des Exemples **18-21** suivants sont obtenus par alkylation réductrice du composé de l'Exemple 1.

### EXEMPLE 18 : Chlorhydrate de 5-(cyclopropylméthyl)-8-phénoxy-2,3,4,5-tétrahydro-1,2,5-benzothiadiazépine 1,1-dioxyde

A une solution du produit de l'Exemple 1 (1,38 mmol) dans 50 ml de CH₂Cl₂, sont ajoutés le cyclopropanecarbaldéhyde (4,17 mmol), l'acide acétique (4,17 mmol) et le triacétoxyborohydrure de sodium (4,17 mmol). Le milieu réactionnel est agité une nuit à température ambiante puis est neutralisé par addition d'une solution à 10 % en NaHCO₃. Après extraction au dichlorométhane, les phases organiques sont rassemblées, lavées (solution saturée en NaCl), séchées (MgSO₄) puis évaporées. Le brut est purifié par chromatographie sur silice (dichlorométhane / méthanol 98 / 2) et le produit est salifié dans l'éthanol par addition d'une solution de HCl 4 N dans le dioxane pour conduire au produit du titre.

### Point de fusion : 62 °C (meringue)

### EXEMPLE 19 : 5-cyclobutyl-8-phénoxy-2,3,4,5-tétrahydro-1,2,5-benzothiadiazépine 1,1-dioxyde

Le mode opératoire est identique à celui de l'Exemple 18 mais la réaction est agitée à 70 °C dans le dichloroéthane en utilisant la cyclobutanone au lieu du cyclopropanecarbaldéhyde. Le brut est purifié par chromatographie sur silice en éluant avec un mélange acétate d'éthyle / cyclohexane 2 / 8 pour conduire, après évaporation au produit du titre.

### Point de fusion : 55 °C (meringue)

### Microanalyse élémentaire :

| | *C* | *H* | *N* | *S* |
|---|---|---|---|---|
| *% théorique* | *62, 77* | *5,85* | *8,13* | *9,31* |
| *% expérimental* | *62,81* | *5,90* | *7,98* | *9,34* |

### EXEMPLE 20 : 5-méthyl-8-phénoxy-2,3,4,5-tétrahydro-1,2,5-benzothiadiazépine 1,1-dioxyde

Le mode opératoire est identique à celui de l'Exemple 18 en utilisant du formaldéhyde aqueux 37 % au lieu du cyclopropanecarbaldéhyde. Le brut est purifié par chromatographie sur silice en éluant avec un mélange dichlorométhane / méthanol 98/2 pour conduire au produit du titre.

### Point de fusion : 128-131 °C

### Microanalyse élémentaire :

| | *C* | *H* | *N* | *S* |
|---|---|---|---|---|
| *% théorique* | *59,19* | *5,30* | *9, 20* | *10,54* |
| *% expérimental* | *59,35* | *5,30* | *9, 07* | *10,52* |

### EXEMPLE 21 : 5-éthyl-8-phénoxy-2,3,4,5-tctrahydro-1,2,5-benzothiadiazépine 1,1-dioxyde

Le mode opératoire est identique à celui de l'Exemple 18 en utilisant de l'acétaldéhyde au lieu du cyclopropanecarbaldéhyde. Le brut est purifié par chromatographie sur silice en éluant avec un mélange dichlorométhane / méthanol 98/2 pour conduire au produit du titre.

### Point de fusion : 138°C

### Microanalyse élémentaire :

| | *C* | *H* | *N* | *S* |
|---|---|---|---|---|
| *% théorique* | *60,36* | *5,70* | *8,80* | *10,07* |
| *% expérimental* | *60, 25* | *5,65* | *8,70* | *10,18* |

### EXEMPLE 22 : 5-cyclopropyl-8-phénoxy-2,3,4,5-tétrahydro-1,2,5-benzothiadiazépine 1,1-dioxyde

Le mode opératoire est identique à celui de l'Exemple 18 mais la réaction est agitée à 70 °C dans le dichloroéthane en utilisant le [(1-éthoxycyclopropyl)oxy]triméthylsilane au lieu du cyclopropanecarbaldéhyde. Le brut est purifié par chromatographie sur silice en éluant avec un mélange cyclohexane / acétate d'éthyle 70 / 30 pour conduire au produit du titre.

### Point de fusion : 119°C

### Microanalyse élémentaire :

| | *C* | *H* | *N* | *S* |
|---|---|---|---|---|
| *% théorique* | *61,80* | *5,49* | *8,48* | *9,71* |
| *% expérimental* | *61,15* | *5,53* | *8, 25* | *9, 60* |

### EXEMPLE 23 : 2,5-diméthyl-8-phénoxy-2,3,4,5-tétrahydro-1,2,5-benzothiadiazépine 1,1-dioxyde

A une suspension du produit de l'Exemple 1 (1,016 mmol) dans 3 ml de diméthylformamide, est ajouté l'hydrure de sodium 60 % dans l'huile minérale (3,05 mmol). Après 10 minutes d'agitation, une solution de iodure de méthyle 2 M dans le méthylterbutyléther (3,05 mmol) est ajoutée goutte à goutte. Le milieu réactionnel est agité une nuit à température ambiante puis est dilué dans de l'eau, extrait par l'acétate d'éthyle, lavé (solution saturée en NaCl), séché (MgSO₄), filtré puis évaporé à sec. Le résidu est chromatographié sur silice en éluant avec un mélange dichlorométhane / acétone pour conduire au produit du titre.

### Point de fusion : 130-131 °C

### Microanalyse élémentaire :

| | *C* | *H* | *N* | *S* |
|---|---|---|---|---|
| *% théorique* | *60,36* | *5,70* | *8,80* | *10,07* |
| *% expérimental* | *60,58* | *5,75* | *8, 64* | *10,31* |

### EXEMPLE 24 : 2-(2-fluoroéthyl)-8-phénoxy-2,3,4,5-tétrahydro-1,2,5-benzothiadiazépine 1,1-dioxyde

Une suspension du produit de l'Exemple 1 (2,06 mmol) dans 100 ml d'acétonitrile est agitée 24 heures en présence de carbonate de césium (4,12 mmol) et de 1-bromo-2-fluoroéthane (4,12 mmol). Le milieu réactionnel est ensuite filtré et le filtrat est évaporé à sec. Le brut est chromatographié sur silice en éluant avec un mélange dichlorométhane / méthanol 98 / 2. Le produit est salifié dans l'éthanol par addition d'une solution de HCl 4 N dans le dioxane pour conduire au produit du titre sous forme d'un chlorhydrate.

### Point de fusion : 95-98 °C

### ETUDE PHARMACOLOGIQUE

### EXEMPLE A : Etude de l'effet des produits sur la dépolarisation membranaire induite par l'AMPA sur des cultures primaires de neurones de rat.

Le test consiste en la mesure *in vitro*, par fluorescence, de la dépolarisation membranaire induite sur des neurones embryonnaires de rat en culture, par l'action conjointe de l'AMPA et du produit testé, en comparaison à l'action de l'AMPA seul. Les cellules du cerveau sont mises en culture et maintenues dans un incubateur de culture cellulaire pendant 18 jours. Après incubation, le milieu de culture est retiré et remplacé par du milieu de chargement en sonde de fluorescence pour mesure du potentiel membranaire (20 µl ; kit de potentiel de membrane de Molecular Devices) et laissées à température ambiante pendant 1 heure. La fluorescence de base des puits est lue (appareil FDSS de Hamamatsu), puis l'AMPA est injecté sur les cellules (20 µl ; gamme de concentration de 3 à 100 µM) et l'action de l'AMPA est mesurée en cinétique. Le produit testé est ensuite introduit dans les puits (20 µl; en gamme de concentration, croisée avec celle de l'AMPA) et l'action du produit est mesurée en cinétique. A l'issue de chacune des 2 périodes de mesure en cinétique, la valeur retenue pour chaque puits est la moyenne de la lecture sur les 15 dernières secondes de la période. On représente les courbes d'effet de l'AMPA aux différentes concentrations de produit. Pour chaque concentration de produit, la valeur retenue est l'aire sous la courbe d'AMPA à cette concentration et l'EC₂ₓ, concentration de produit qui double le potentiel membranaire induit par l'AMPA, est calculée.

Les composés de l'invention potentialisent fortement les effets excitateurs de l'AMPA. A titre d'exemple, le composé de l'Exemple 1 présente une EC₂ₓ de 25 µM.

### EXEMPLE B : Reconnaissance d'objet chez la souris CD1

Le test de reconnaissance d'objet (Behav. Brain Res., 1988, 31, 47-59) est basé sur l'activité exploratoire spontanée de l'animal et possède les caractéristiques de la mémoire épisodique chez l'Homme. Sensible au vieillissement (Eur. J. Pharm. 1997, 325, 173-180), ainsi qu'aux dysfonctionnements cholinergiques (Pharm. Biochem. Behav., 1996, 53(2), 277-283), ce test de mémoire est fondé sur l'exploration différentielle de 2 objets de forme assez similaire, l'un familier, l'autre nouveau. La procédure expérimentale, adaptée à la souris CD1, comporte 3 phases qui se déroulent dans la même enceinte expérimentale. Pendant la 1^{ère} phase qui dure 40 minutes, les souris sont habituées à l'environnement. Pendant la 2^{e} phase, qui a lieu le lendemain, un objet est placé dans l'enceinte et la souris est libre de l'explorer. Lorsque cette exploration atteint une durée de 20 secondes, la souris est retirée de l'enceinte. Au cours de la 3^{e} phase (5 minutes), 24 heures plus tard, le même objet est présenté (il acquiert le statut d'objet « familier »), ainsi qu'un nouvel objet. La durée d'exploration, exprimée en secondes, de chacun des deux objets, est chronométrée. Les animaux témoins, ayant reçu préalablement le véhicule par voie orale 60 minutes avant chacune des 3 phases, explorent pendant une durée équivalente l'objet « familier » et l'objet « nouveau », ce qui signifie l'oubli de l'objet déjà présenté. Les animaux ayant reçu un composé facilitateur mnémocognitif, explorent de façon préférentielle l'objet nouveau, ce qui signifie le maintien du souvenir de l'objet déjà présenté.

Testés selon le protocole de l'Exemple B, les composés de formule (I) selon l'invention se sont révélés efficaces dans l'amélioration de la mémorisation. Par exemple, les résultats obtenus avec le composé de l'Exemple 1 de la présente invention montrent une exploration significativement plus importante de l'objet nouveau par rapport à l'objet familier aux doses de 1 et 3 mg/kg, PO.

### EXEMPLE C : Effet des produits sur le courant induit par le NMDA sur des ovocytes de Xenopus laevis injectés avec de l'ARNₘ poly(A⁺) de cortex de rat

Des enregistrements électrophysiologiques sont réalisés sur des ovocytes de *Xenopus laevis* injectés avec de l'ARNₘ poly(A⁺) de cortex de rat, et exprimant entre autre des récepteurs glutamatergiques de type NMDA, dans une chambre d'enregistrement en plexiglas perfusée en continu avec une solution d'OR2 ne contenant pas de magnésium (bloquant l'ouverture du canal du récepteur NMDA) et à température ambiante. Le courant entrant induit par une application de NMDA est enregistré à un potentiel de repos de -60 mV en utilisant une technique standard de potentiel imposé à deux électrodes. Le NMDA (3.10⁻⁴ M) est appliqué avec la solution de perfusion en présence de 3.10⁻⁵ M de glycine pendant 30 secondes toutes les 5 minutes avec une vitesse de perfusion constante de 3 ml/min. L'amplitude du courant induit par le NMDA est mesurée au pic du courant. Les produits testés sont appliqués à des doses croissantes sur le même ovocyte dans la solution de perfusion 45 secondes avant, 30 secondes pendant et 30 secondes après l'application de 3.10⁻⁴M de NMDA en présence de 3.10⁻⁵ M de glycine toutes les 5 minutes. L'amplitude du courant induit par le NMDA en présence de produit est normalisée et exprimée en pourcentage de celui induit sur le même ovocyte en absence de produit, correspondant au 100 % de la réponse. L'IC₅₀ correspondant à la concentration de produit inhibant de 50 % le courant induit par le NMDA est déterminée par régression non linéaire avec un modèle de concentration-réponse de forme sigmoïdale à pente variable.

Les composés de l'invention inhibent fortement les effets du NMDA. A titre d'exemple, le composé de l'Exemple 1 présente une IC₅₀ de 9 µM.

### EXEMPLE D : Composition pharmaceutique

| | |
|---|---|
| Formule de préparation pour 1000 comprimés dosés à 10 mg en 8-phénoxy-2,3,4,5-tétrahydro-1,2,5-benzothiadiazépine 1,1-dioxyde (Exemple 1) | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I) : 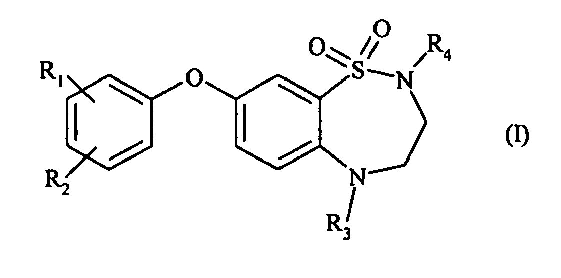 dans laquelle :
➢ R₁ et R₂, identiques ou différents, représentent chacun, un atome d'hydrogène ; un atome d'halogène ; un groupement alkyle (C₁-C₆) linéaire ou ramifié non-substitué ou substitué par un ou plusieurs atomes d'halogène ; un groupement alkoxy (C₁-C₆) linéaire ou ramifié ; un groupement alkylthio (C₁-C₆) linéaire ou ramifié ; un groupement alkoxycarbonyle (C₁-C₆) linéaire ou ramifié ; un groupement carboxy ; un groupement acyle (C₁-C₆) linéaire ou ramifié ; un groupement hydroxy ; un groupement hydroxyalkyle (C₁-C₆) linéaire ou ramifié ; un groupement cyano ; un groupement nitro ; un groupement amino non-substitué ou substitué par un ou plusieurs groupements alkyle (C₁-C₆) linéaire ou ramifié ; un groupement amino substitué par un groupement acyle (C₁-C₆) linéaire ou ramifié ; un groupement aminocarbonyle non-substitué ou substitué par un ou plusieurs groupements alkyle (C₁-C₆) linéaire ou ramifié ; un groupement aminosulfonyle non-substitué ou substitué par un ou plusieurs groupements alkyle (C₁-C₆) linéaire ou ramifié ; un groupement alkyl(C₁-C₆)sulfonylaminoalkyle (C₁-C₆) linéaire ou ramifié ; un groupement N-hydroxycarboximidamide ou un groupement benzyloxy,
➢ R₃ représente un atome d'hydrogène ou un groupement alkyle (C₁-C₆) linéaire ou ramifié, cycloalkyle (C₃-C₈) ou cycloalkyl(C₃-C₈)alkyle (C₁-C₆) linéaire ou ramifié,
➢ R₄ représente un atome d'hydrogène ou un groupement alkyle (C₁-C₆) linéaire ou ramifié non-substitué ou substitué par un ou plusieurs atomes d'halogène,
leurs isomères optiques et de position ainsi que leurs sels d'addition à un acide ou à une base pharmaceutiquement acceptable.

2. Composés de formule (I) selon la revendication 1, **caractérisés en ce que** le groupement R₁ représente un atome d'hydrogène ou un groupement hydroxy, un groupement hydroxyméthyle, un groupement éthoxycarbonyle, un groupement amino ou un groupement *N*-méthylaminocarbonyle.

3. Composés de formule (I) selon la revendication 1 ou 2, **caractérisés en ce que** le groupement R₁ se trouve en position méta ou para.

4. Composés de formule (I) selon l'une quelconque des revendications 1 à 3, **caractérisés en ce que** les groupements R₂ et R₄ représentent un atome d'hydrogène.

5. Composés de formule (I) selon l'une quelconque des revendications 1 à 4, **caractérisés en ce que** le groupement R₃ représente un atome d'hydrogène ou un groupement méthyle.

6. Composés de formule (I) selon la revendication 1, qui sont le :
• 8-phénoxy-2,3,4,5-tétrahydro-1,2,5-benzothiadiazépine 1,1-dioxyde ;
• 3-[(1,1-dioxydo-2,3,4,5-tétrahydro-1,2,5-benzothiadiazépin-8-yl)oxy]benzoate d'éthyle ;
• 3-[(1,1-dioxydo-2,3,4,5-tétrahydro-1,2,5-benzothiadiazépin-8-yl)oxy]-*N-*méthyl benzamide ;
• {3-[(1,1-dioxydo-2,3,4,5-tétrahydro-1,2,5-benzothiadiazépin-8-yl)oxy]phényl} méthanol ;
• 4-[(1,1-dioxydo-2,3,4,5-tétrahydro-1,2,5-benzothiadiazépin-8-yl)oxy]phénol ;
• 4-[(5-méthyl-1,1-dioxydo-2,3,4,5-tétrahydro-1,2,5-benzothiadiazépin-8-yl)oxy] phénol ;
• 4-[(1,1-dioxydo-2,3,4,5-tétrahydro-1,2,5-benzothiadiazépin-8-yl)oxy]aniline,
ainsi que leurs sels d'addition à un acide ou à une base pharmaceutiquement acceptable.

7. Procédé de préparation des composés de formule (I) selon la revendication 1, **caractérisé en ce que** l'on utilise comme produit de départ un composé de formule (II) : 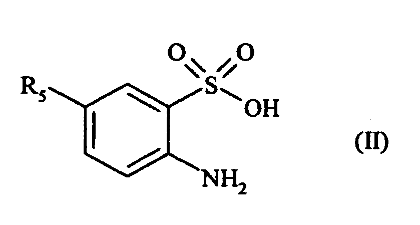 dans laquelle R₅ représente un groupement alkoxy (C₁-C₆) linéaire ou ramifié,
qui est mis en réaction avec le chlorure de thionyle en présence de diméthylformamide, pour conduire au composé de formule (III) : 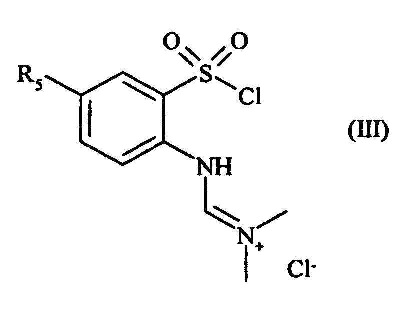 dans laquelle R₅ est tel que défini précédemment,
qui subit alors l'action de la 2-chloroéthylamine en milieu basique, pour conduire au composé de formule (IV) : 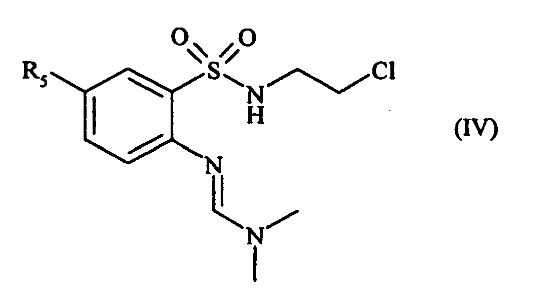 dans laquelle R₅ est tel que défini précédemment,
qui, après déprotection en milieu acide, est ensuite cyclisé pour conduire au composé de formule (V) : 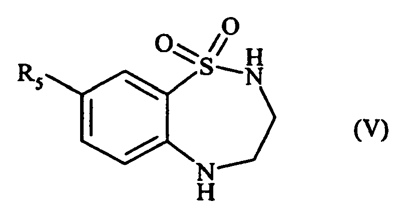 dans laquelle R₅ est tel que défini précédemment,
qui subit alors l'action du tribromure de bore, pour conduire au composé de formule (VI) : 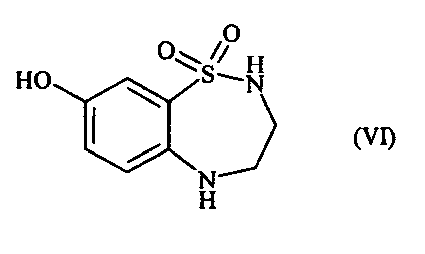 que l'on fait réagir avec un dérivé d'acide boronique de formule (VII) : 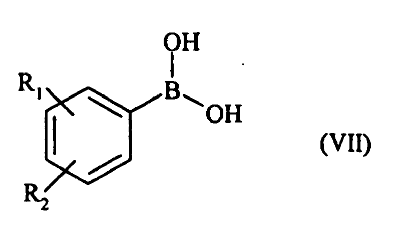 dans laquelle R₁ et R₂ ont la même signification que dans la formule (I),
pour conduire au composé de formule (I/a), cas particulier des composés de formule (I) : 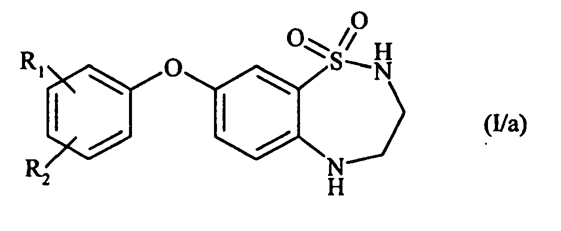 dans laquelle R₁ et R₂ sont tels que définis précédemment,
une variante dans la préparation du composé de formule (I/a) consistant, une fois l'étape de couplage sur le composé de formule (VI) réalisée, en l'utilisation des réactions classiques de chimie afin de modifier, dans un deuxième temps, les substituants du dérivé de l'acide boronique,
composé de formule (I/a) qui peut alors subir, si nécessaire :
■ **soit** une double alkylation sur les azotes en positions 2 et 5, par action d'une base forte en présence d'un agent d'alkylation R'-X dans lequel R' représente un groupement alkyle (C₁-C₆) linéaire ou ramifié et X représente un atome d'halogène,
pour conduire au composé de formule (I/b), cas particulier des composés de formule (1) : 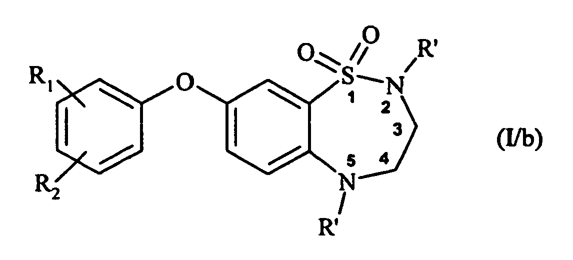 dans laquelle R₁, R₂ et R' sont tels que définis précédemment,
■ **soit** une alkylation sur l'azote en position 2, par action d'une base en présence d'un agent d'alkylation R'₄-X dans lequel R'₄ représente un groupement alkyle (C₁-C₆) linéaire ou ramifié substitué ou non-substitué par un ou plusieurs atomes d'halogène et X représente un atome d'halogène,
pour conduire au composé de formule (I/c), cas particulier des composés de formule (I) : 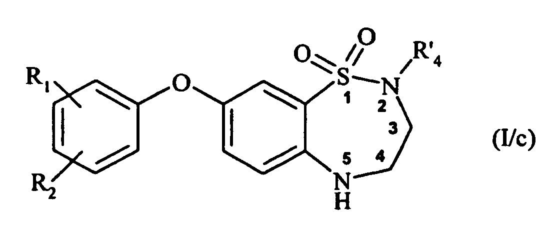 dans laquelle R₁, R₂ et R'₄ sont tels que définis précédemment,
qui peut, éventuellement, subir une alkylation sur l'azote en position 5, par action d'une base en présence d'un agent d'alkylation R'₃-X dans lequel R'₃ représente un groupement alkyle (C₁-C₆) linéaire ou ramifié, cycloalkyle (C₃-C₈) ou cycloalkyl(C₃-C₈)alkyle (C₁-C₆) linéaire ou ramifié, et X représente un atome d'halogène,
pour conduire au composé de formule (I/d) : 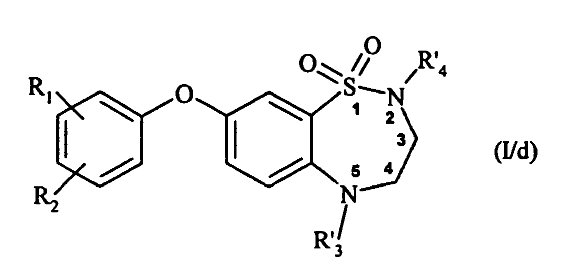 dans laquelle R₁, R₂, R'₃ et R'₄ sont tels que définis précédemment,
■ **soit** une alkylation sur l'azote en position 5, par une réaction d'amination réductrice avec un agent réducteur, tel que le triacétoxyborohydrure ou le cyanoborohydrure de sodium, en présence :
- soit du [(1-éthoxycyclopropyl)oxy] triméthylsilane,
- soit d'un composé de formule (VIII) :
R"₃-CHO (VIII)
dans laquelle R"₃ représente un atome d'hydrogène ou un groupement alkyle (C₁-C₅) linéaire ou ramifié, un groupement cycloalkyle (C₃-C₈) ou cycloalkyl(C₃-C₈)alkyle (C₁-C₅) linéaire ou ramifié,
- soit d'un composé de formule (IX) : 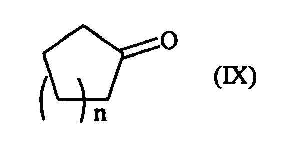 dans laquelle 0 ≤ n ≤ 4,
pour conduire au composé de formule (I/e), cas particulier des composés de formule (I) : 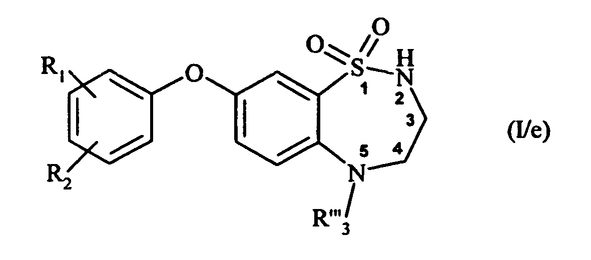 dans laquelle R"'₃ représente un groupement alkyle (C₁-C₆) linéaire ou ramifié, cycloalkyle (C₃-C₈), ou cycloalkyl(C₃-C₈)alkyle (C₁-C₆) linéaire ou ramifié, et R₁ et R₂ sont tels que définis précédemment,
qui peut, éventuellement, subir une alkylation sur l'azote en position 2, par action d'une base en présence d'un agent d'alkylation R"₄-X dans lequel R"₄ représente un groupement alkyle (C₁-C₆) linéaire ou ramifié non-substitué ou substitué par un ou plusieurs atomes d'halogène et X représente un atome d'halogène,
pour conduire au composé de formule (I/f) : 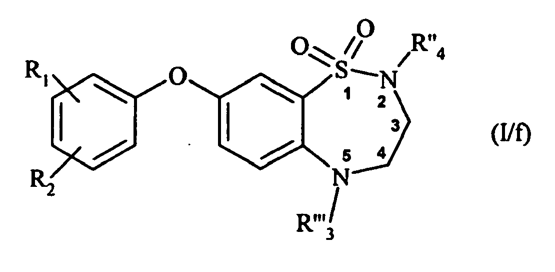 dans laquelle R₁, R₂, R"'₃ et R"₄ sont tels que définis précédemment, les composés de formule (I/a) à (I/f), qui constituent l'ensemble des composés de formule (I), peuvent être ensuite purifiés selon une technique classique de séparation, que l'on transforme, si on le souhaite en ses sels d'addition à un acide ou à une base pharmaceutiquement acceptable et dont on sépare éventuellement les isomères optiques et de position, s'ils existent, selon une technique classique de séparation.

8. Composé de formule (VI) selon la revendication 7 : 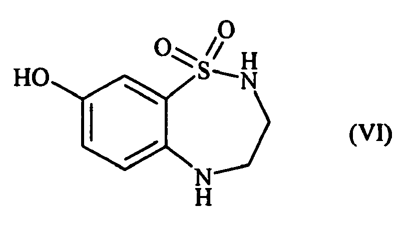 **caractérisé en ce qu'**il est utile en tant qu'intermédiaire de synthèse des composés de formule (I).

9. Compositions pharmaceutiques contenant comme principe actif un composé selon l'une quelconque des revendications 1 à 6, en combinaison avec un ou plusieurs véhicules inertes, non toxiques et pharmaceutiquement acceptables.

10. Compositions pharmaceutiques selon la revendication 9 utiles pour la fabrication de médicaments utiles en tant que modulateurs du récepteur AMPA et antagonistes du récepteur NMDA.

11. Compositions pharmaceutiques selon la revendication 9 utiles pour la fabrication de médicaments utiles pour le traitement ou la prévention des maladies neurodégénératives progressives, de la maladie d'Alzheimer, de la maladie de Parkinson, de la maladie de Pick, de la chorée d'Huntington, de la maladie de Korsakoff, de la schizophrénie, des maladies neurodégénératives aiguës, des démences frontales et sous-corticales, des démences vasculaires, de l'épilepsie, des accidents vasculaires cérébraux ainsi que des états dépressifs et anxieux.

## Claims

1. Compounds of formula (I): 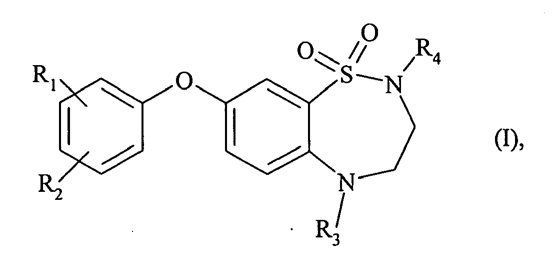 wherein:
➢ R₁ and R₂, which may be the same or different, each represent a hydrogen atom; a halogen atom; a linear or branched (C₁-C₆)alkyl group which is unsubstituted or substituted by one or more halogen atoms; a linear or branched (C₁-C₆)alkoxy group; a linear or branched (C₁-C₆)alkylthio group; a linear or branched (C₁-C₆)alkoxycarbonyl group; a carboxy group; a linear or branched (C₁-C₆)acyl group; a hydroxy group; a linear or branched (C₁-C₆)hydroxyalkyl group; a cyano group; a nitro group; an amino group which is unsubstituted or substituted by one or more linear or branched (C₁-C₆)alkyl groups; an amino group which is substituted by a linear or branched (C₁-C₆)acyl group; an aminocarbonyl group which is unsubstituted or substituted by one or more linear or branched (C₁-C₆)alkyl groups; an aminosulphonyl group which is unsubstituted or substituted by one or more linear or branched (C₁-C₆)alkyl groups; a (C₁-C₆)alkylsulphonylamino-(C₁-C₆)alkyl group in which the alkyl moieties are linear or branched; an N-hydroxycarboximidamide group or a benzyloxy group,
➢ R₃ represents a hydrogen atom or a linear or branched (C₁-C₆)alkyl group, a (C₃-C₈)cycloalkyl group or a (C₃-C₈)cycloalkyl-(C₁-C₆)alkyl group in which the alkyl moiety is linear or branched,
➢ R₄ represents a hydrogen atom or a linear or branched (C₁-C₆)alkyl group which is unsubstituted or substituted by one or more halogen atoms,
their optical and positional isomers, and also addition salts thereof with a pharmaceutically acceptable acid or base.

2. Compounds of formula (I) according to claim 1, **characterised in that** the group R₁ represents a hydrogen atom or a hydroxy group, a hydroxymethyl group, an ethoxycarbonyl group, an amino group or an *N-*methylaminocarbonyl group.

3. Compounds of formula (I) according to either claim 1 or claim 2, **characterised in that** the group R₁ is in the meta or para position.

4. Compounds of formula (I) according to any one of claims 1 to 3, **characterised in that** the groups R₂ and R₄ represent hydrogen atoms.

5. Compounds of formula (I) according to any one of claims 1 to 4, **characterised in that** the group R₃ represents a hydrogen atom or a methyl group.

6. Compounds of formula (I) according to claim 1, which are:
• 8-phenoxy-2,3,4,5-tetrahydro-1,2,5-benzothiadiazepine 1,1-dioxide;
• ethyl 3-[(1,1-dioxido-2,3,4,5-tetrahydro-1,2,5-benzothiadiazepin-8-yl)oxy]-benzoate;
• 3-[(1,1-dioxido-2,3,4,5-tetrahydro-1,2,5-benzothiadiazepin-8-yl)oxy]-*N-*methyl-benzamide;
• {3-[(1,1-dioxido-2,3,4,5-tetrahydro-1,2,5-benzothiadiazepin-8-yl)oxy]phenyl}-methanol;
• 4-[(1,1-dioxido-2,3,4,5-tetrahydro-1,2,5-benzothiadiazepin-8-yl)oxy]phenol;
• 4-[(5-methyl-1,1-dioxido-2,3,4,5-tetrahydro-1,2,5-benzothiadiazepin-8-yl)oxy]-phenol;
• 4-[(1,1-dioxido-2,3,4,5-tetrahydro-1,2,5-benzothiadiazepin-8-yl)oxy]aniline,
and also addition salts thereof with a pharmaceutically acceptable acid or base.

7. Process for the preparation of compounds of formula (I) according to claim 1, **characterised in that** there is used as starting material a compound of formula (II): 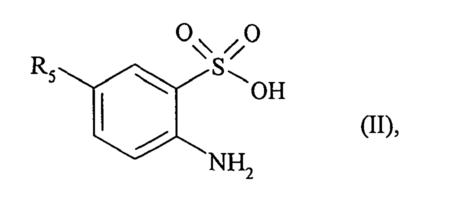 wherein R₅ represents a linear or branched (C₁-C₆)alkoxy group,
which is reacted with thionyl chloride in the presence of dimethylformamide to yield the compound of formula (III): 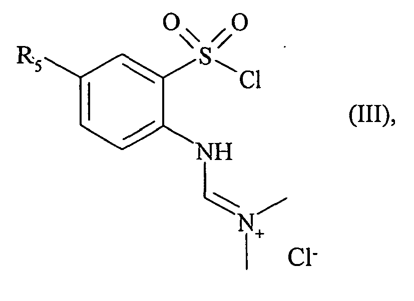 wherein R₅ is as defined hereinbefore,
which is then subjected to the action of 2-chloroethylamine in a basic medium to yield the compound of formula (IV): 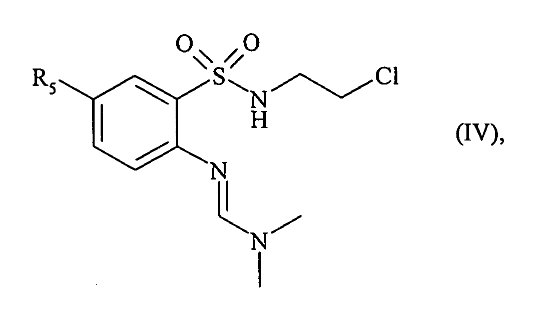 wherein R₅ is as defined hereinbefore,
which, after deprotection in an acid medium, is then cyclised to yield the compound of formula (V): 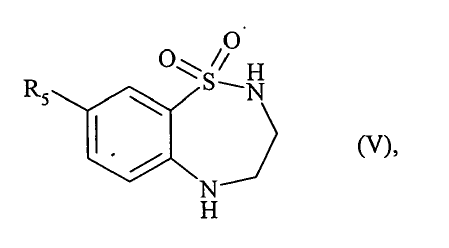 wherein R₅ is as defined hereinbefore,
which is then subjected to the action of boron tribromide to yield the compound of formula (VI): 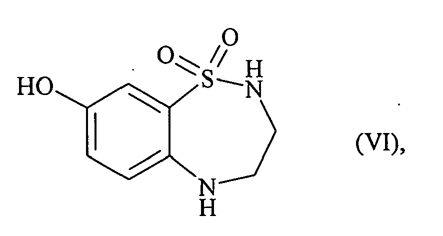 which is reacted with a boronic acid compound of formula (VII): 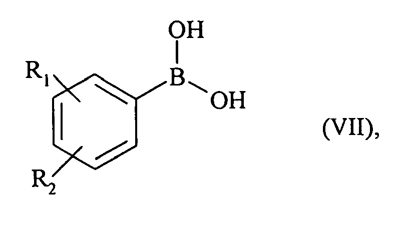 wherein R₁ and R₂ are as defined for formula (I),
to yield the compound of formula (I/a), a particular case of the compounds of formula (I): 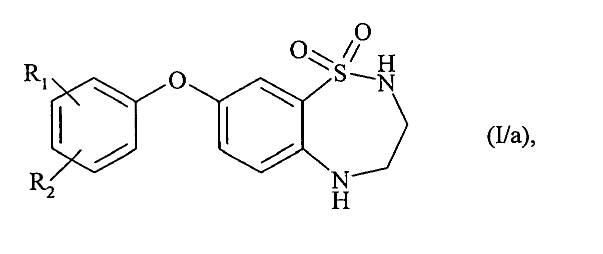 wherein R₁ and R₂ are as defined hereinbefore,
a variant in the preparation of the compound of formula (I/a) consisting of using - once the step of coupling with the compound of formula (VI) has been carried out - conventional chemical reactions to subsequently modify the substituents of the boronic acid compound,
which compound of formula (I/a) may then, if required, be subjected to:
■ **either** double alkylation on the nitrogen atoms in positions 2 and 5, by action of a strong base in the presence of an alkylating agent R'-X wherein R' represents a linear or branched (C₁-C₆)alkyl group and X represents a halogen atom,
to yield the compound of formula (I/b), a particular case of the compounds of formula (I): 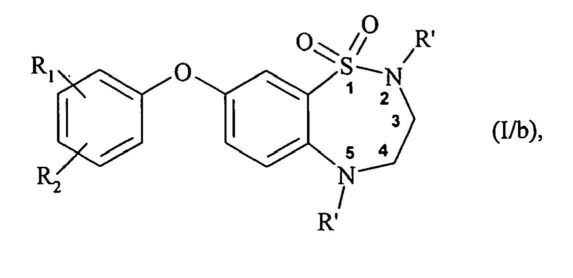 wherein R₁, R₂ and R' are as defined hereinbefore,
■ **or** alkylation on the nitrogen atom in position 2, by action of a base in the presence of an alkylating agent R'₄-X wherein R'₄ represents a linear or branched (C₁-C₆)alkyl group which is unsubstituted or substituted by one or more halogen atoms and X represents a halogen atom,
to yield the compound of formula (I/c), a particular case of the compounds of formula (I): 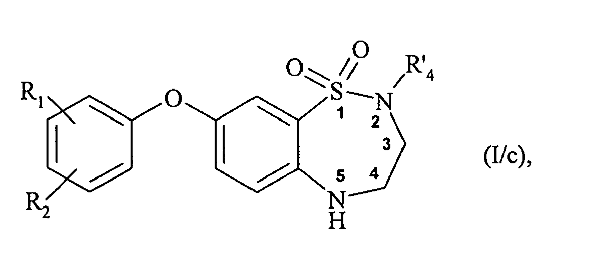 wherein R₁, R₂ and R'₄ are as defined hereinbefore,
which may optionally be subjected to alkylation on the nitrogen atom in position 5, by action of a base in the presence of an alkylating agent R'₃-X wherein R'₃ represents a linear or branched (C₁-C₆)alkyl group, a (C₃-C₈)cycloalkyl group or a (C₃-C₈)cycloalkyl-(C₁-C₆)alkyl group in which the alkyl moiety is linear or branched and X represents a halogen atom,
to yield the compound of formula (I/d): 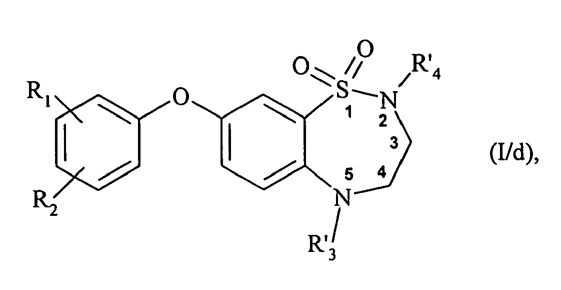 wherein R₁, R₂, R'₃ and R'₄ are as defined hereinbefore,
**■ or alternatively** alkylation on the nitrogen atom in position 5, by means of a reductive amination reaction using a reducing agent such as sodium triacetoxyborohydride or cyanoborohydride in the presence of:
- either [(1-ethoxycyclopropyl)oxy]trimethylsilane,
- or a compound of formula (VIII):
R"₃-CHO (VIII),
wherein R"₃ represents a hydrogen atom or a linear or branched (C₁-C₅)alkyl group, a (C₃-C₈)cycloalkyl group or a (C₃-C₈)cycloalkyl-(C₁-C₅)alkyl group in which the alkyl moiety is linear or branched,
- or alternatively a compound of formula (IX): 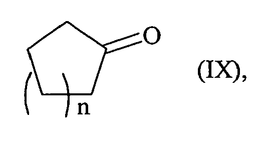 wherein 0 ≤ n ≤ 4, to yield the compound of formula (I/e), a particular case of the compounds of formula (I): 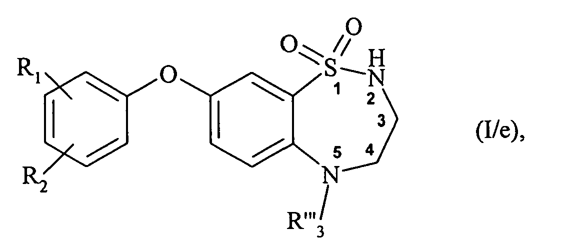 wherein R"'₃ represents a linear or branched (C₁-C₆)alkyl group, a (C₃-C₈)cycloalkyl group or a (C₃-C₈)cycloalkyl-(C₁-C₆)alkyl group in which the alkyl moiety is linear or branched, and R₁ and R₂ are as defined hereinbefore,
which may optionally be subjected to alkylation on the nitrogen atom in position 2, by action of a base in the presence of an alkylating agent R"₄-X wherein R"₄ represents a linear or branched (C₁-C₆)alkyl group which is unsubstituted or substituted by one or more halogen atoms and X represents a halogen atom,
to yield the compound of formula (I/f): 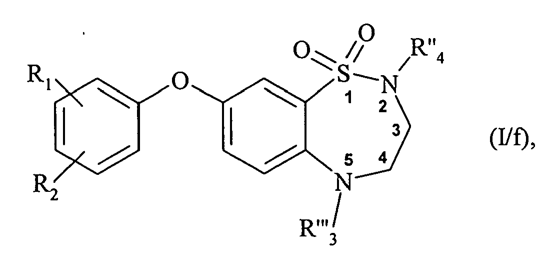 wherein R₁, R₂, R"'₃ and R"₄ are as defined hereinbefore,
which compounds of formulae (I/a) to (I/f), which constitute the totality of the compounds of formula (I), may then be purified according to a conventional separation technique, are converted, if desired, into their addition salts with a pharmaceutically acceptable acid or base and are separated, where appropriate, into their optical and positional isomers, if they exist, according to a conventional separation technique.

8. Compound of formula (VI) according to claim 7: 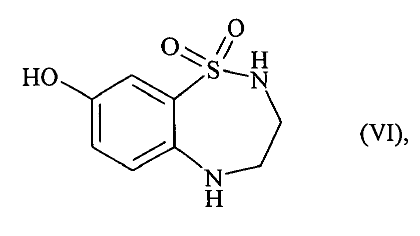 **characterised in that** it is for use as an intermediate in the synthesis of compounds of formula (I).

9. Pharmaceutical compositions comprising as active ingredient a compound according to any one of claims 1 to 6, in combination with one or more pharmaceutically acceptable, non-toxic, inert carriers.

10. Pharmaceutical compositions according to claim 9 for use in the manufacture of medicaments for use as modulators of the AMPA receptor and antagonists of the NMDA receptor.

11. Pharmaceutical compositions according to claim 9 for use in the manufacture of medicaments for use in the treatment or prevention of progressive neurodegenerative diseases, Alzheimer's disease, Parkinson's disease, Pick's disease, Huntington's chorea, Korsakoff's disease, schizophrenia, acute neurodegenerative diseases, frontal lobe and subcortical dementias, vascular dementias, epilepsy, cerebral vascular accidents and also depressive and anxious states.

## Patentansprüche

1. Verbindungen der Formel (I): 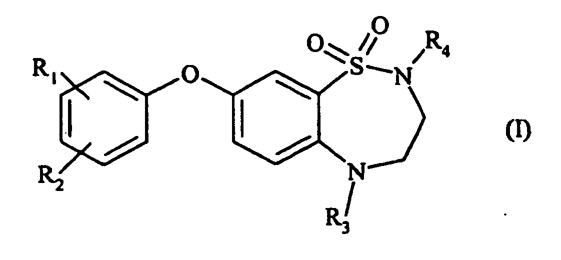 in der:
➢ R₁ und R₂, die identisch oder verschieden sind, jeweils ein Wasserstoffatom; ein Halogenatom; eine geradkettige oder verzweigte nichtsubstituierte oder durch ein oder mehrere Halogenatome substituierte (C₁-C₆)-Alkylgruppe; eine geradkettige oder verzweigte (C₁-C₆)-Alkoxygruppe; eine geradkettige oder verzweigte (C₁-C₆)-Alkylthiogruppe; eine geradkettige oder verzweigte (C₁-C₆)-Alkoxycarbonylgruppe; eine Carboxygruppe; eine geradkettige oder verzweigte (C₁-C₆)-Acylgruppe; eine Hydroxygruppe; eine geradkettige oder verzweigte (C₁-C₆)-Hydroxyalkylgruppe; eine Cyanogruppe; eine Nitrogruppe; eine nichtsubstituierte oder durch ein oder mehrere geradkettige oder verzweigte (C₁-C₆)-Alkylgruppen substituierte Aminogruppe; eine durch eine geradkettige oder verzweigte (C₁-C₆)-Acylgruppe substituierte Aminogruppe; eine nichtsubstituierte oder durch eine oder mehrere geradkettige oder verzweigte (C₁-C₆)-Alkylgruppen substituierte Aminocarbonylgruppe; eine nichtsubstituierte oder durch eine oder mehrere geradkettige oder verzweigte (C₁-C₆)-Alkylgruppen substituierte Aminosulfonylgruppe; eine geradkettige oder verzweigte (C₁-C₆)-Alkyl-sulfonylamino-(C₁-C₆)-alkylgruppe; eine N-Hydroxycarboximidamidgruppe oder eine Benzyloxygruppe bedeuten,
➢ R₃ ein Wasserstoffatom oder eine geradkettige oder verzweigte (C₁-C₆)-Alkylgruppe, (C₃-C₈)-Cycloalkylgruppe oder eine geradkettige oder verzweigte (C₃-C₈)-Cycloalkyl-(C₁-C₆)-alkylgruppe bedeutet,
➢ R₄ ein Wasserstoffatom oder eine geradkettige oder verzweigte, nichtsubstituierte oder durch ein oder mehrere Halogenatome substituierte (C₁-C₆)-Alkylgruppe bedeutet, deren optische und Positions-Isomeren sowie deren Additionssalze mit einer pharmazeutisch annehmbaren Säure oder Base.

2. Verbindungen der Formel (I) nach Anspruch 1, **dadurch gekennzeichnet, dass** die Gruppe R₁ ein Wasserstoffatom, eine Hydroxygruppe, eine Hydroxymethylgruppe, eine Ethoxycarbonylgruppe, eine Aminogruppe oder eine *N-*Methylaminocarbonylgruppe bedeutet.

3. Verbindungen der Formel (I) nach Anspruch 1 oder 2, **dadurch gekennzeichnet, dass** die Gruppe R₁ in der meta- oder para-Stellung steht.

4. Verbindungen der Formel (I) nach einem der Ansprüche 1 bis 3, **dadurch gekennzeichnet, dass** die Gruppen R₂ und R₄ ein Wasserstoffatom bedeuten.

5. Verbindungen der Formel (I) nach einem der Ansprüche 1 bis 4, **dadurch gekennzeichnet, dass** die Gruppe R₃ ein Wasserstoffatom oder eine Methylgruppe bedeutet.

6. Verbindungen der Formel (I) nach Anspruch 1, nämlich:
• 8-Phenoxy-2,3,4,5-tetrahydro-1,2,5-benzothiadiazepin-1,1-dioxid;
• 3-[(1,1-Dioxido-2,3,4,5-tetrahydro-1,2,5-benzothiadiazepin-8-yl)-oxy]-benzoesäureethylester;
• 3-[(1,1-Dioxido-2,3,4,5-tetrahydro-1,2,5-benzothiadiazepin-8-yl-oxy]-N methylbenzamid;
• {3-[(1,1-Dioxido-2,3,4,5-tetrahydro-1,2,5-benzothiadiazepin-8-yl)-oxy]-phenyl}-methanol;
• 4-[(1,1-Dioxido-2,3,4,5-tetrahydro-1,2,5-benzothiadiazepin-8-yl)-oxy]-phenol;
• 4-[(5-Methyl-1,1-dioxido-2,3,4,5-tetrahydro-1,2,5-benzothiadiazepin-8-yl)-oxy]-phenol;
• 4-[(1,1-Dioxido-2,3,4,5-tetrahydro-1,2,5-benzothiadiazepin-8-yl)-oxy]-anilin sowie deren Additionssalze mit einer pharmazeutisch annehmbaren Säure oder Base.

7. Verfahren zur Herstellung der Verbindungen der Formel (I) nach Anspruch 1, **dadurch gekennzeichnet, dass** man als Ausgangsprodukt eine Verbindung der Formel (II) verwendet: 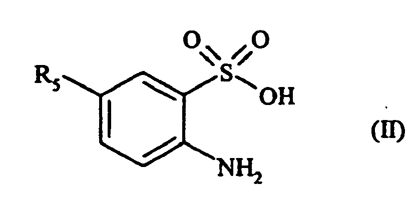 in der R₅ eine geradkettige oder verzweigte (C₁-C₆)-Alkoxygruppe darstellt,
welche man mit Thionylchlorid in Gegenwart von Dimethylformamid umsetzt zur Bildung der Verbindung der Formel (III): 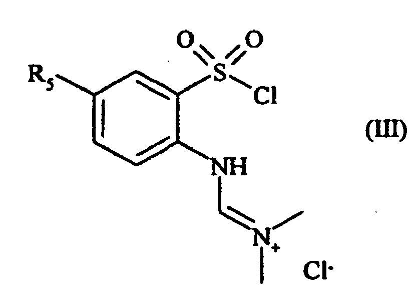 in der R₅ die oben angegebene Bedeutung besitzt,
welche der Einwirkung von 2-Chlorethylamin in basischem Medium unterworfen wird zur Bildung der Verbindung der Formel (IV): 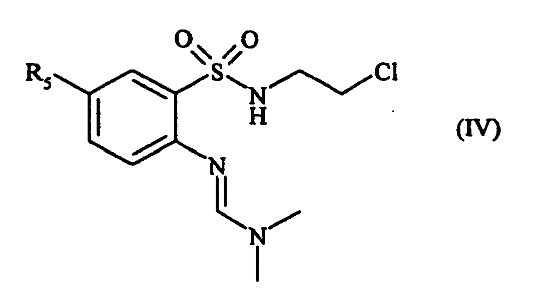 in der R₅ die oben angegebene Bedeutung besitzt,
welche nach der Abspaltung der Schutzgruppe in saurem Medium anschließend cyclisiert wird zur Bildung der Verbindung der Formel (V): 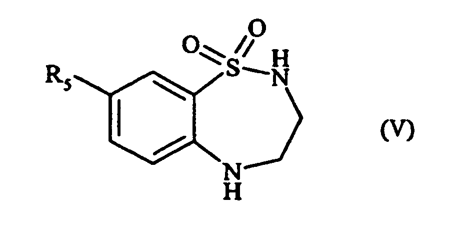 in der R₅ die oben angegebene Bedeutung besitzt,
die dann der Einwirkung von Bortribromid unterworfen wird zur Bildung der Verbindung der Formel (VI): 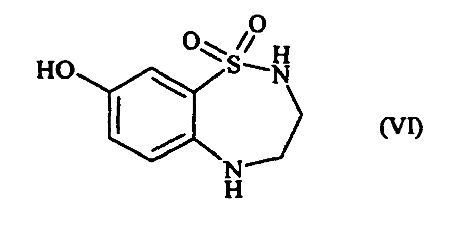 welche man mit einem Boronsäurederivat der Formel (VII): 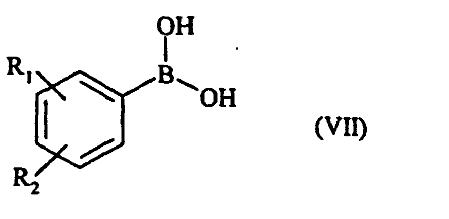 umsetzt, in der R₁ und R₂ die bezüglich der Formel (I) angegebenen Bedeutungen besitzen,
zur Bildung der Verbindung der Formel (I/a), einem Sonderfall der Verbindungen der Formel (I): 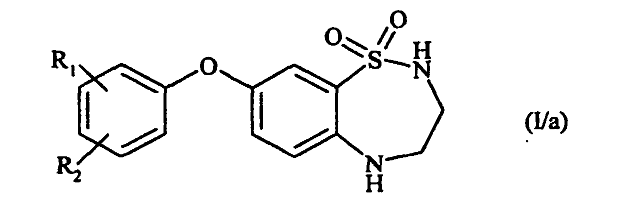 in der R₁ und R₂ die oben angegebenen Bedeutungen besitzen,
wobei eine Variante für die Herstellung der Verbindung der Formel (I/a) darin besteht, dass man nach der Durchführung der Stufe der Kupplung der Verbindung der Formel (VI) in einer zweiten Stufe die Substituenten des Boronsäurederivats unter Anwendung klassischer Reaktionen der Chemie modifiziert,
welche Verbindung der Formel (I/a) dann erforderlichenfalls:
■ **entweder** einer doppelten Alkylierung an den Stickstoffatomen in den Positionen 2 und 5 unterworfen wird durch Einwirkung einer starken Base in Gegenwart eines Alkylierungsmittels R'-X, worin R' eine geradkettige oder verzweigte (C₁-C₆)-Alkylgruppe und X ein Halogenatom bedeuten,
zur Bildung der Verbindung der Formel (I/b), einem Sonderfall der Verbindungen der Formel (I): 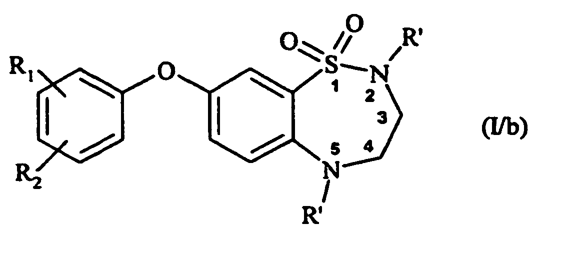 in der R₁, R₂ und R' die oben angegebenen Bedeutungen besitzen,
■ **oder** einer Alkylierung am Stickstoffatom in der 2-Stellung unterwirft durch Einwirkung einer Base in Gegenwart eines Alkylierungsmittels R'₄-X, worin R'₄ eine geradkettige oder verzweigte, nichtsubstituierte oder durch ein oder mehrere Halogenatome substituierte (C₁-C₆)-Alkylgruppe und X ein Halogenatom bedeuten,
zur Bildung der Verbindung der Formel (I/c), einem Sonderfall der Verbindungen der Formel (I): 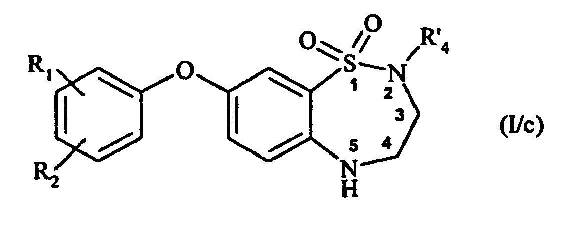 in der R₁, R₂ und R'₄ die oben angegebenen Bedeutungen besitzen,
welche gegebenenfalls einer Alkylierung am Stickstoffatom in der 5-Stellung unterworfen wird durch Einwirkung einer Base in Gegenwart eines Alkylierungsmittels R'₃-X, worin R'₃ eine geradkettige oder verzweigte (C₁-C₆)-Alkylgruppe, eine (C₃-C₈)-Cycloalkylgruppe oder eine geradkettige oder verzweigte (C₃-C₈)-Cycloalkyl-(C₁-C₆)-alkylgruppe und X ein Halogenatom bedeuten,
zur Bildung der Verbindung der Formel (I/d): 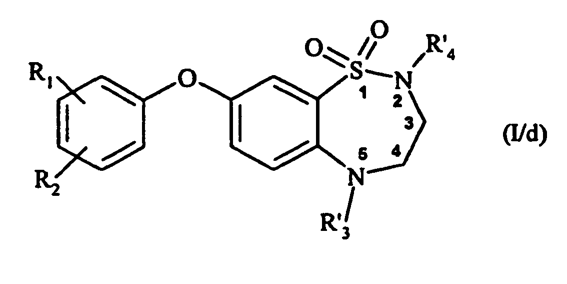 in der R₁, R₂, R'₃ und R'₄ die oben angegebenen Bedeutungen besitzen,
■ **oder** einer Alkylierung an dem Stickstoffatom in der 5-Stellung unterwirft durch eine reduktierende Aminierungsreaktion mit einem Reduktionsmittel, wie Triacetoxyborhydrid oder Natriumcyanborhydrid, in Gegenwart von:
- entweder [(1-Ethoxycyclopropyl)-oxy]-trimethylsilan,
- oder einer Verbindung der Formel (VIII):
R"₃ - CHO (VIII)
in der R"₃ ein Wasserstoffatom oder eine geradkettige oder verzweigte (C₁-C₅)-Alkylgruppe, eine (C₃-C₈)-Cycloalkylgruppe oder eine geradkettige oder verzweigte (C₃-C₈)-Cycloalkyl-(C₁-C₅)-alkylgruppe bedeutet,
- oder einer Verbindung der Formel (IX): 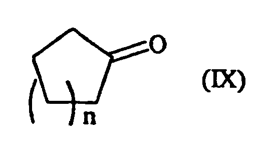 in der 0 ≤ n ≤ 4 bedeutet,
zur Bildung der Verbindung der Formel (I/e), einem Sonderfall der Verbindungen der Formel (I): 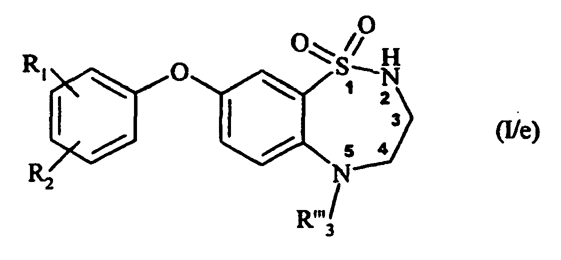 in der R"'₃ eine geradkettige oder verzweigte (C₁-C₆)-Alkylgruppe, eine (C₃-C₈)-Cycloalkylgruppe oder eine geradkettige oder verzweigte (C₃-C₈)-Cycloalkyl-(C₁-C₆)-alkylgruppe bedeutet und R₁ und R₂ die oben angegebenen Bedeutungen besitzen,
welche man gegebenenfalls einer Alkylierung am Stickstoffatom in der 2-Stellung unterwerfen kann durch Einwirkung einer Base in Gegenwart eines Alkylierungsmittels R"₄-X, worin R"₄ eine geradkettige oder verzweigte, nichtsubstituierte oder durch ein oder mehrere Halogenatome substituierte (C₁-C₆)-Alkylgruppe und X ein Halogenatom bedeuten,
zur Bildung der Verbindung der Formel (I/f): 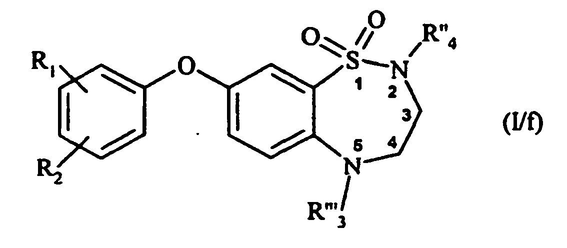 in der R₁, R₂, R"'₃ und R"₄ die oben angegebenen Bedeutungen besitzen,
welche Verbindungen der Formeln (I/a) bis (I/f), welche die Gesamtheit der Verbindungen der Formel (I) bilden, anschließend mit Hilfe einer klassischen Trennmethode gereinigt werden können, welche man gewünschtenfalls in ihre Additionssalze mit einer pharmazeutisch annehmbaren Säure oder Base überführt und welche man gegebenenfalls mit Hilfe einer klassischen Trennungsmethode in ihre optischen und Positions-Isomeren auftrennt, falls diese existieren.

8. Verbindung der Formel (VI) nach Anspruch 7: 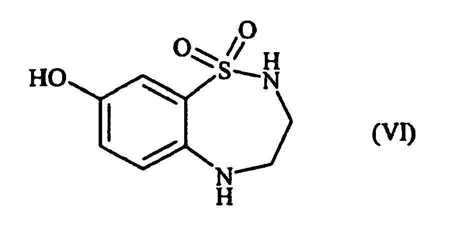 **dadurch gekennzeichnet, dass** sie als Zwischenprodukt der Synthese der Verbindungen der Formel (I) nützlich ist.

9. Pharmazeutische Zubereitungen enthaltend als Wirkstoff eine Verbindung nach einem der Ansprüche 1 bis 6 in Kombination mit einem oder mehreren inerten, nichttoxischen und pharmazeutisch annehmbaren Hilfsstoffen.

10. Pharmazeutische Zubereitungen nach Anspruch 9 nützlich für die Herstellung von Arzneimitteln, die nützlich sind als Modulatoren des Rezeptors AMPA und als Antagonisten des Rezeptors NMDA.

11. Pharmazeutische Zubereitungen nach Anspruch 9 nützlich für die Herstellung von Arzneimitteln, die nützlich sind zur Behandlung oder Vorbeugung von progressiven neurodegenerativen Erkrankungen, der Alzheimerschen Krankheit, der Parkinsonschen Krankheit, der Pickschen Krankheit, der Huntington Chorea, der Korsakoff-Krankheit, der Schizophrenie, von akuten neurodegenerativen Erkrankungen, frontalen und subkortikalen Demenzen, vaskulären Demenzen, der Epilepsie, Schlaganfällen sowie Depressions- und Angstzuständen.
